# EUROPÄISCHE PATENTANMELDUNG

(11) **EP 3 034 604 A1**
(43) Veröffentlichungstag der Anmeldung: **22.06.2016**
(21) Anmeldenummer: 15199617.0
(22) Anmeldetag: 11.12.2015
(51) Int. Cl.: C12M 1/107, C12M 1/00, C12M 1/34

(54) **VERFAHREN UND VORRICHTUNG ZUR RÜCKLÖSUNG UND WIEDERGEWINNUNG VON STRUVIT**

(30) Priorität: 19.12.2014 DE 102014119193
(71) Anmelder: Holm, Niels Christian, 23479 Hille (DE)
(72) Erfinder: Holm, Niels Christian, 23479 Hille (DE); Sierig, Sarah, 32427 Minden (DE); Wunder, Kerstin, 32479 Hille (DE)
(74) Vertreter: Lobemeier, Martin Landolf

(57) **Zusammenfassung**

Verfahren zur Herstellung einer Struvit-Ablagerungen in Rohrleitungen (17, 18) beseitigenden Reinigungsflüssigkeit, mit den Schritten: Erzeugen eines Kondensats (2) aus Rohbiogas (3), Zuführen eines Gases zum Kondensat zum Verdrängen des im Kondensat gelösten Kohlendioxids bei gleichzeitigem Ausgasen des im Kondensat gelösten Ammoniaks, Sieden des Kondensats (2) zum Ausgasen des im Kondensat (2) gelösten Ammoniaks, Zuführen von Kohlendioxid (10) zum von Kohlendioxid und Ammoniak weitgehend befreiten Kondensat bis das Kondensat einen sauren pH-Wert aufweist, und Bereitstellen des Kondensats mit einem sauren pH-Wert als Struvit-Ablagerungen in Rohrleitungen beseitigende Reinigungsflüssigkeit.

## Beschreibung

Die Erfindung betrifft ein Verfahren zur Rücklösung und Wiedergewinnung von Struvit (Magnesium-Ammonium-Phosphat: Mg²⁺NH₄⁺PO₄³⁻) aus Ablagerungen an Rohrleitungsinnenwänden aller Art.

Im Rahmen der anaeroben Vergärung fester und flüssiger Biomassen aller Art, z. B. biologische Abfallstoffe, wie Schlämme und Gülle, oder Pflanzen, wie Mais und Zuckerrüben, wird organisches Material zu Biogas vergoren. Bei diesem Prozess werden erhebliche Mengen an anorganischen Salzen freigesetzt, u. a. Stickstoff in Form von Ammonium (NH₄⁺), Phosphor in Form von Phosphat (PO₄³⁻) und Magnesium (Mg²⁺).

Falls diese Gärprozesse (z. B. in Faulbehältern von Kläranlagen und in Fermentern von Biogasanlagen) so hohe Mengen an diesen drei Salzionen freisetzen, dass das Löslichkeitsprodukt des entsprechenden Salzes überschritten wird, fallen die über das Löslichkeitsprodukt hinausgehend freigesetzten Salzionen als kristallines Struvit aus. Dieses ausgefallene Struvit ist dann Teil des Faulschlammes.

Im Faulschlamm richtet das Struvit keinen Schaden an. Die Struvitkristalle neigen allerdings dazu, sich auf Oberflächen abzulagern und eine massive Kristallschicht zu bilden.

Die Struvitablagerungen auf Innenwänden von Rohrleitungen (als Verbindung zwischen Faulbehälter und anderen Behältern) sind für den Anlagenbetrieb entsprechender Anlagen sehr nachteilig: Über die Zeit wachsen die Rohrleitungen zu und die resultierenden Verengungen schränken die Durchsatzleistungen immer mehr ein. Im schlimmsten Fall sind die Rohrleitungen nach einigen Jahren vollständig verstopft.

Eine Vielzahl von Gründen hat in den letzten Jahrzehnten zu einer Verschärfung der Struvit - Problematik geführt. Dazu gehören beispielsweise:
- immer effektivere Gärverfahren, mit denen die Freisetzung immer größerer Mengen an Salzen einhergeht;
- die Verwendung von Biomassen, die besonders hohe Konzentrationen an Stickstoff, Magnesium und Phosphor enthalten, z. B. Gülle;
- der Aufschluss und die Rezyklierung aufgeschlossener Faulschlämme zur Erhöhung der Faulgasproduktion, ebenfalls einhergehend mit einer Anreicherung der Salzionen.

Daher wurden unterschiedliche Verfahren entwickelt und eingesetzt, um entweder die StruvitBildung zu verhindern oder zu reduzieren (Magnetverfahren) oder um in regelmäßigen Abständen die Innenwände der Rohrleitungen von Struvit freizuwaschen.

Das Magnetverfahren kann die Struvitbildung zwar nicht verhindern, führt aber dazu, dass das Struvit in eher feinkristalliner Form ausfällt. Das Zuwachsen der Rohrleitungsinnenwände wird dadurch zwar verzögert, nicht aber verhindert.

Bei den Verfahren zur Freiwaschung der Rohrleitung werden den Rohrleitungen zugeführte Säuren (z. B. Salzsäure oder Essigsäure) eingesetzt. Genutzt wird dabei die Tatsache, dass das Löslichkeitsprodukt von Struvit bei sauren pH-Werten sehr viel größer ist als bei alkalischen pH-Werten und somit unter sauren Bedingungen wieder in Lösung geht.

Diese Verfahren sind allerdings kostenintensiv, da hierfür externe Säuren verbraucht werden müssen. Die genannten Säuren müssen auch relativ stark mit Wasser verdünnt werden, da der pH-Wert der Säuren im Rohrleitungssystem zwischen 3 bis 5 liegen sollte. Zu niedrige pH-Werte können die Rohrleitungen beschädigen. Da zur Herstellung der sauren Reinigungsflüssigkeit Ab- und Spülwässer aufgrund ihrer hohen Ionenfracht ungeeignet sind, ist auch der Wasserverbrauch in Form von Trinkwasser relativ hoch.

Aufgabe der Erfindung ist es daher, ein Verfahren zum Entfernen von Struvit-Ablagerungen aus Rohrleitungen zu schaffen, dass ressourcenschonend, ökonomisch und effizient ist, sodass keine externen Säuren verwendet werden müssen und der Wasserverbrauch reduziert werden kann.

Erfindungsgemäß wird diese Aufgabe durch das Verfahren zur Herstellung einer Struvit-Ablagerungen in Rohrleitungen beseitigenden Reinigungsflüssigkeit mit den Merkmalen von Anspruch 1, durch das Verfahren zur Reinigung einer Struvit-Ablagerungen aufweisenden Rohrleitung mit den Merkmalen von Anspruch 6 und durch die Vorrichtung mit den Merkmalen von Anspruch 11 gelöst. Die abhängigen Ansprüche geben jeweils bevorzugte Ausführungsbeispiele an.

Grundgedanke der Erfindung ist es, das bei der Trocknung von Rohbiogas anfallende Kondensat zur Herstellung einer Struvit-Ablagerungen beseitigenden Reinigungslösung zu verwenden. Als Ersatz für das üblicherweise verwendete Trinkwasser wird das aus dem Biogas auskondensierte Kondenswasser verwendet und durch Nutzung des im Rohbiogas oder dem getrockneten Biogas vorhandenen Kohlendioxids zur Herstellung von Kohlensäure anstelle von externen Säuren nutzbar gemacht.

Auf allen Anlagen, die Biogas/Faulgas produzieren, fällt in erheblichen Mengen Kondenswasser an, das bei der Abkühlung des Biogases entsteht. Rohbiogas enthält ca. 50 bis 100 g Kondenswasser pro m³ i.N. Biogas, je nach mesophilen (ca. 40 °C) oder thermophilen (ca. 52 °C) Betriebsbedingungen. Bei einer Anlage mit einer Produktion von 10.000 m³ i.N./Tag Biogas würden somit ca. 0,5 - 1,0 m³ Kondenswasser/Tag anfallen.

Dieses Kondenswasser wird üblicherweise in die Faul-/Biogas-/Lagerbehälter für Gärreste oder in den Wasserbehandlungsteil von Kläranlagen zurückgefördert und so entsorgt. Über alternative Verwendungsformen ist nie etwas berichtet worden (bzw. den Autoren dieser Schrift nicht bekannt), weshalb in der Literatur auch keine Angaben über die chemische Zusammensetzung des Kondenswassers in Abhängigkeit von der chemischen Zusammensetzung der Gärreste zu finden sind.

Eigene Untersuchungen haben ergeben, dass der Ammoniak/Ammonium (NH₃/NH₄⁺) Gehalt unterschiedlicher Kondenswässer verschiedener Biogasanlagen nur ca. 20 % der entsprechenden Werte in den Gärresten beträgt. Liegt dort die Ammoniumkonzentration bei z. B. 3.500 mg/l, beträgt die Konzentration im dazugehörigen Kondenswasser also ca. 700 mg/l.

Diese Korrelation ist nicht vollständig linear, sondern hängt auch vom pH-Wert ab. Für Anwendungszwecke kann aber vereinfachend von 20 % ausgegangen werden.

Andere anorganische Salzionen (außer Karbonate: CO₃²⁻ und HCO₃⁻) sind im in Biogasanlagen anfallenden Kondenswasser nicht oder nur in Spuren vorhanden, Phosphatmessungen haben beispielsweise sehr niedrige Werte von ca. 1 mg/l ergeben. Die pH-Werte aller vom Anmelder bisher untersuchten Kondenswässer lagen bei pH 6,9 bis pH 7,1.

Das Kondenswasser entspricht somit ungefähr destilliertem Wasser mit erheblichen Ammoniakkonzentrationen und erheblichen CO₂-Konzentrationen (aus den 20 bis 50 % im Rohbiogas enthaltenen CO₂). Kohlendioxid (CO₂) ist ein Gas, das sehr leicht aus dem Kondenswasser entfernt werden kann. Ammoniak bzw. Ammoniakwasser mit Ammoniak-Konzentrationen wie im Kondenswasser von Biogas ist eine Flüssigkeit mit einem Siedepunkt des Ammoniaks bei ca. 70 °C, also deutlich unter dem Siedepunkt von Wasser.

Das erfindungsgemäße Verfahren zur Herstellung einer Struvit-Ablagerungen in Rohrleitungen beseitigenden Reinigungsflüssigkeit, weist also die folgenden Schritte auf: a. Erzeugen eines Kondensats aus Rohbiogas, b. Zuführen eines Gases zum Kondensat zum Verdrängen des im Kondensat gelösten Kohlendioxids, wodurch der pH-Wert erhöht und das Ammoniak leichter ausgegast werden kann, c. darauffolgend oder bevorzugt gleichzeitig Sieden des Kondensats zum Ausgasen des im Kondensat gelösten Ammoniaks, d. Zuführen von Kohlendioxid zum von Kohlendioxid und Ammoniak weitgehend befreiten Kondensat bis das Kondensat einen sauren pH-Wert, und e. Bereitstellen des Kondensats mit einem sauren pH-Wert als Struvit-Ablagerungen in Rohrleitungen beseitigende Reinigungsflüssigkeit.

"Weitgehend von Kohlendioxid und Ammoniak befreit" bedeutet in diesem Zusammenhang insbesondere, dass die Ammonium/Ammoniak-Konzentration gegenüber dem Ursprungskondensat um ≤ 70 % reduziert worden ist.

Der saure pH-Wert der Reinigungsflüssigkeit liegt also in einem Bereich pH <7. Bevorzugt liegt der saure pH-Wert der Reinigungsflüssigkeit in einem Bereich von pH 3,0 bis 5,0. Besonders bevorzugt liegt der saure pH-Wert der Reinigungsflüssigkeit in einem Bereich von pH 4,5 bis 5,0.

Insbesondere ist der pH-Wert der Reinigungsflüssigkeit einerseits so niedrig zu wählen, dass die Struvit-Ablagerungen effektiv beseitigt werden können. Andererseits sollte der pH-Wert der Reinigungsflüssigkeit so hoch gewählt werden, dass die Anlagenkomponenten, insbesondere die Rohrleitungen, nicht von der Säure angegriffen werden.

Das in Schritt b) zugeführte Gas kann Luft oder Methan sein. Es kommt im Wesentlichen nur darauf an, dass die Konzentration an Kohlendioxid in der Gasphase so gewählt ist, dass das im Kondensat gelöste Kohlendioxid aus dem Kondensat ausgasen kann.

Die Schritte b) und c) werden aus energetischen und Zeiteinsparungsgründen bevorzugt zeitgleich ausgeführt.

Das Kohlendioxid in Schritt d) wird bevorzugt in Form von Rohbiogas oder aus Rohbiogas gewonnenem CO₂ zugeführt.

Das Zuführen des Gases im Schritt b) und/oder das Zuführen von Kohlendioxid in Schritt d) wird bevorzugt durch Einleiten des Gases bzw. des Kohlendioxids in das Kondensat vorgenommen, damit möglichst schnell ein Äquilibrium zwischen Gasphase und Flüssigkeitsphase im Kondensat erreicht wird.

Die Erfindung betrifft auch ein Verfahren zur Reinigung einer Struvit-Ablagerungen aufweisenden Rohrleitung, mit den Schritten: Erzeugen einer Struvit-Ablagerungen in Rohrleitungen beseitigenden Reinigungsflüssigkeit nach einem der vorhergehenden Ansprüche, und Einleiten der Reinigungsflüssigkeit in die Rohrleitung und Einwirken lassen der Reinigungsflüssigkeit auf die Struvit-Ablagerungen bis die Struvit-Ablagerungen in der Reinigungsflüssigkeit rückgelöst sind.

Die Reinigungsflüssigkeit wird bevorzugt nach Einwirken auf die Rohrleitungen die Struvit-Ablagerungen in Lösung aufweisen und mit diesen in einen Lagerbehälter (20) ausgeleitet. Besonders bevorzugt wird die die Struvit-Ablagerungen in Lösung aufweisende Reinigungsflüssigkeit jedoch in einen Rückgewinnungsbehälter ausgeleitet und das in dieser Flüssigkeit enthaltende Kohlendioxid wird durch Versetzen dieser Flüssigkeit mit einem Gas unter Erhöhen des pH-Werts zum Ausfällen des Struvits ausgetrieben. Durch diese Maßnahme fällt das Struvit aus der Lösung aus und kann beispielsweise durch eine trichterförmige Abfuhreinrichtung entnommen und anderweitig verwendet werden.

Die Struvit depletierte Reinigungsflüssigkeit hingegen wird besonders bevorzugt zur Herstellung der Struvit-Ablagerungen in Rohrleitungen beseitigenden Reinigungsflüssigkeit gemeinsam mit dem Kondensat oder anstelle des Kondensats behandelt, sodass ein Kreislauf für die Reinigungsflüssigkeit entsteht.

Wie weiter unten im Detail erklärt, wird die die Struvit-Ablagerungen in Lösung aufweisende Reinigungsflüssigkeit bevorzugt in einen Rückgewinnungsbehälter ausgeleitet, solange der pH-Wert der Reinigungsflüssigkeit einen vorbestimmten pH-Wert überschreitet.

Schließlich betrifft die Erfindung auch eine Biogasanlage mit einer ein Kondensat erzeugenden Trocknungseinrichtung zur Trocknung von Rohbiogas und einer für Struvit-Ablagerungen anfälligen Rohrleitung, wobei die Biogasanlage erfindungsgemäß einen Behälter zur Aufbereitung des Kondensats zu einer Struvit-Ablagerungen beseitigenden Reinigungsflüssigkeit, mit einer Heizeinrichtung zum Sieden des Kondensats, einer pH-Sonde zur Messung des pH-Werts des Kondensats, und wenigstens einer dem Behälter ein Gas zuführenden Leitung aufweist, wobei der Behälter zur Aufbereitung des Kondensats mittels Rohrleitungen mit der Trocknungseinrichtung und der für Struvit-Ablagerungen anfälligen Rohrleitung verbunden ist.

Bevorzugt ist die für Struvit-Ablagerungen anfällige Rohrleitung mit einem die in der Reinigungsflüssigkeit rückgelösten Struvit-Ablagerungen aufnehmenden Lagerbehälter verbunden.

Weiter bevorzugt weist die Biogasanlage einen zur Rückgewinnung von Struvit aus der Reinigungsflüssigkeit eingerichteten Rückgewinnungsbehälter auf, der mit der für Struvit-Ablagerungen anfälligen Rohrleitung und dem Behälter verbunden ist, Dabei weist der Rückgewinnungsbehälter eine Belüftungseinrichtung zum Versetzen der gelöste Struvit-Ablagerungen aufweisenden Reinigungsflüssigkeit mit einem Gas zum Erhöhen des pH-Werts zum Ausfällen des Struvits auf

Insbesondere besitzt der Rückgewinnungsbehälter einen trichterförmigen Abschnitt zur Aufnahme und Abfuhr des aus der Reinigungsflüssigkeit ausgefällten Struvits.

Die Erfindung wird anhand von in den Zeichnungen dargestellten, besonders bevorzugt ausgestalteten Ausführungsbeispielen näher erläutert. Es zeigen:
- Fig. 1: eine schematische Seitenansicht der Ausführung gemäß Anspruch 1 für die Struvit-Rücklösung nach der Erfindung; und
- Fig. 2: eine schematische Seitenansicht einer besonders bevorzugt ausgestalteten Ausführungsbeispiels für die Struvit-Rückausfällung nach der Erfindung

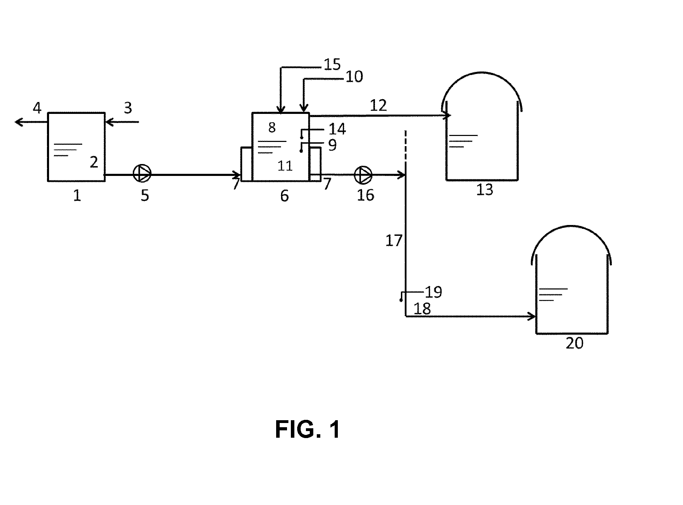

Fig. 1 zeigt eine schematische Seitenansicht der Ausführung gemäß Anspruch 1 einer Vorrichtung nach dieser Erfindung.

Das Rohbiogas 3 durchfließt einen Kondensatschacht 1 bevor es nach Austritt 4 zum Endverbraucher oder weiteren Behandlung geleitet wird. Im Kondensatschacht sammelt sich das Kondenswasser 2. Mittels einer Pumpe 5 wird das Kondenswasser nicht zurück in die Anlage, sondern in einen besonderen Behälter 6 gefördert, in dem folgende sequenzielle Behandlungsschritte erfolgen:
Zuerst erfolgt über 10 die Zufuhr eines Gases, das CO₂ aus dem Kondenswasser verdrängt/strippt. Bei diesem Gas handelt es sich um reine Luft oder alternativ reines Methangas, das bei einer Biogasaufbereitung zur Verfügung steht. Dieses Gas wird entweder nur der Gasphase 8 zugeführt, oder alternativ, um den Prozess der CO₂-Strippung zu beschleunigen, in die Flüssigphase 11 hineingeblasen. Die verdrängte Gasmenge wird bevorzugter Weise über die Leitung 12 in die Gasphase eines Gärbehälters 13 gefördert/entsorgt.

Durch Einblasen des Gases in das Kondenswasser wird der CO₂-Stripprozess stark beschleunigt und ist nach weit weniger als einer Stunde nahezu abgeschlossen, erkennbar an einem schnellen pH-Wert Anstieg im Kondenswasser auf ca. 8. Dieser Prozess wird bevorzugter Weise mittels einer pH-Sonde 14 geregelt/gesteuert.

Die pH-Wert Erhöhung verlagert das NH₃/NH₄⁺-Gleichgewicht in Richtung des Ammoniaks (NH₃), das nachfolgend auch gestrippt werden soll (NH₄⁺ ist ein Ion, das nicht gestrippt werden kann).

Parallel dazu oder erst anschließend erfolgt die Erwärmung des Kondenswassers auf eine Temperatur oberhalb des Siedepunktes von Ammoniakwasser, z. B. 80 °C mittels einer Wärmezufuhreinheit 7, die extern oder intern angeordnet werden kann; zur Kontrolle / Regelung der Temperatur ist dieser Behälter mit einer Temperaturmessung 9 ausgerüstet.

Nachfolgend wird die Gaszufuhr fortgesetzt und führt zu einer Ammoniak-Strippung aus dem Kondenswasser, erkennbar an einen pH-Wert Abfall auf deutlich unter pH 7. Erfolgt das Einblasen des Gases in das erwärmte Kondenswasser, wird der Ammoniak-Stripprozess stark beschleunigt und hat nach ca. 2 Stunden zu einer Ammoniak/Ammonium Reduktion von ca. 96 % geführt. Auch dieser Prozessschritt wird bevorzugter Weise mit der pH-Sonde 14 geregelt.

Nach diesen drei sequenziellen Prozessschritten beträgt die Temperatur des Kondenswassers ca. 80 °C, es ist weitgehend von CO₂ und Ammoniak befreit und hat einen pH-Wert von ca. pH 6,5. Damit ist es chemisch nahezu identisch mit destilliertem Wasser und hat somit eine extrem geringe Pufferkapazität.

Der vierte Prozessschritt beginnt bevorzugt mit der Zufuhr von reinem CO₂ Gas über eine separate Zufuhrleitung 15 oder die vorhandene Gasleitung 10, wenn dieses im Rahmen einer Gasaufbereitung auf der Anlage vorhanden ist. Alternativ wird Rohbiogas mit einem CO₂-Gehalt von 20 bis 50 % verwendet. Bevorzugter Weise erfolgt wiederum das Hineinblasen des CO₂-Gases in das Kondenswasser, wodurch die Kohlensäurebildung stark beschleunigt wird und nach ca. 1 Stunde weitgehend abgeschlossen ist, resultierend in einer Reduktion des pH-Wertes auf besonders bevorzugt pH 4,5 bis pH 5.

Dieses Kohlensäurewasser hat besonders bevorzugt einen pH-Wert von ca. 4,5 bis 5, ist weitgehend frei von Phosphat- und Magnesium-Ionen und hat nur sehr geringen Ammonium-Konzentrationen. Diese Flüssigkeit eignet sich hervorragend für die Rücklösung von Struvit-Inkrustierungen auf den Innenwänden von Rohrleitungen.

Dafür wird dieses Wasser mittels einer Pumpe 16 in entsprechende Rohrleitungen 17 hineingefördert mit einer Einwirkzeit von 1 bis 3 Stunden. Nach ca. 3 Stunden ist die Struvit Rücklösung nahezu abgeschlossen. Versuche im eigenen Labor haben Rücklöseraten von ca. 1,2 g/l Phosphat nach ca. 3 Stunden ergeben, das entspricht ca. 1,7 g/l Struvit. Im Rahmen dieser Struvit-Rücklösung steigt der pH-Wert auf ca. pH 6,5 an.

Am Ende der frei zu spülenden Rohrleitungsstrecke 18 wird bevorzugter Weise eine pH-Sonde 19 installiert, die den Abschluss der Struvit-Rücklösung anzeigt: Anfänglich wird der pH-Wert bei ca. pH 6,5 liegen (siehe oben). Nach Abschluss der Rücklösung fällt der pH-Wert auf den Ursprungswert von ca. 4,5 - 5 des CO₂-begasten Kondensatwassers zurück, weil keine Struvit-Ablagerungen mehr zurückgelöst werden.

Damit ist der Rücklöseprozess abgeschlossen und die Spülung kann beendet werden. Das gesamte Spülwasser (mit dem zurückgelösten Struvit) kann im System verbleiben und wird mit dem "normalen" Gärmaterial beispielsweise in einen Lagerbehälter 20 gefördert.

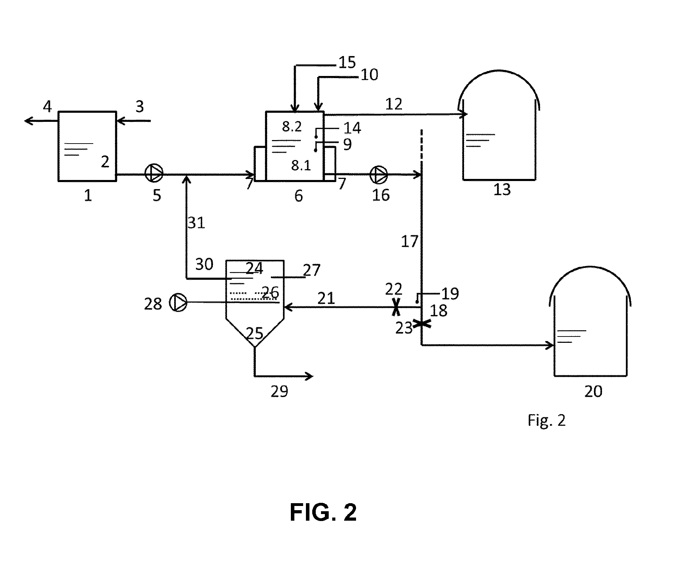

Nach der in Fig. 2 dargestellten, alternativen Ausführungsform erfolgt die Rückgewinnung des Struvits dagegen aus dem System. Das Struvit ist in Kohlensäurewasser aufgelöst worden, und die Kohlensäure kann aus diesem Wasser ausgeblasen werden.

Dafür wird an der pH-Wert Messstelle 19 ein Rohrleitungsbypass 21 eingebaut, betreibbar über die beiden Schieber 22 und 23. Der Betrieb über diesen Bypass erfolgt in einen separaten Rückgewinnungsbehälter 24 hinein und zwar so lange wie die pH-Sonde 19 über einen signifikant über 4,5 bis 5 liegenden pH-Wert, also einem pH-Wert, der mindestens 1 oberhalb des pH-Wertes der CO₂-Reinigungsflüssigkeit liegt, anzeigt, dass in dieser Wasserfraktion bedeutende Mengen an rückgelöstem Struvit vorhanden ist.

Der Rückgewinnungsbehälter 24 ist mit einem Trichter 25 versehen sowie mit einem deutlich über dem Trichterbereich installierten Belüftungseinrichtung 26. Nach Füllung dieses Behälters mit Wasser mit hohen Konzentrationen an Struvit-Ionen 27 erfolgt die Belüftung mit einem weitgehend Kohlendioxid-freien Gas, beispielsweise Luft oder Methan, mit Hilfe eines Gebläses 28. Dadurch wird die Kohlensäure als CO₂ aus dem Wasser hinausgeblasen (gestrippt), was zu einem starken pH-Wert Anstieg auf ca. pH 8,5 führt. Dieser pH-Wert-Anstieg bewirkt die Rückausfällung des Struvits, das nach Abstellen der Belüftung in die Trichterspitze sedimentiert. Laborversuche haben ergeben, dass mittels dieses Verfahrens zur Rückausfällung ca. 80 % wieder kristallin ausgefällt wird. Die verbleibenden ca. 20 % verbleiben in Lösung.

Der Struvit-Schlamm kann aus der Trichterspitze abgezogen werden 29 und direkt oder nach weiteren Veredelungsschritten (wie z. B. Trocknung) vermarktet werden. Ein Teil oder der ganze Klarwasserüberstand 30 mit den verbleibenden ca. 20 % Struvit in Lösung kann wiederholt für die Struvit-Rücklösung verwendet werden 31 oder wird in den Gärprozess zurückgefördert.

## Patentansprüche

1. Verfahren zur Herstellung einer Struvit-Ablagerungen in Rohrleitungen (17, 18) beseitigenden Reinigungsflüssigkeit, mit den Schritten:
a. Erzeugen eines Kondensats (2) aus Rohbiogas (3),
b. Zuführen eines Gases zum Kondensat zum Verdrängen des im Kondensat gelösten Kohlendioxids bei gleichzeitigem Ausgasen des im Kondensat gelösten Ammoniaks,
c. Sieden des Kondensats (2) zum Ausgasen des im Kondensat (2) gelösten Ammoniaks,
d. Zuführen von Kohlendioxid (10) zum von Kohlendioxid und Ammoniak weitgehend befreiten Kondensat bis das Kondensat einen sauren pH-Wert aufweist, und
e. Bereitstellen des Kondensats mit einem sauren pH-Wert als Struvit-Ablagerungen in Rohrleitungen beseitigende Reinigungsflüssigkeit.

2. Verfahren nach Anspruch 1, **dadurch gekennzeichnet, dass** der saure pH-Wert in einem Bereich von 3,0 bis 5,0 liegt.

3. Verfahren nach einem der vorhergehenden Ansprüche, **dadurch gekennzeichnet, dass** das in Schritt b) zugeführte Gas Luft oder Methan ist.

4. Verfahren nach einem der vorhergehenden Ansprüche, **dadurch gekennzeichnet, dass** die Schritte b) und c) zeitgleich ausgeführt werden.

5. Verfahren nach einem der vorhergehenden Ansprüche, **dadurch gekennzeichnet, dass** das Kohlendioxid in Schritt d) in Form von Rohbiogas oder aus Rohbiogas gewonnenem Kohlendioxid (15) zugeführt wird.

6. Verfahren nach einem der vorhergehenden Ansprüche, **dadurch gekennzeichnet, dass** das Zuführen des Gases in Schritt c) und/oder das Zuführen von Kohlendioxid in Schritt d) durch Einleiten des Gases bzw. des Kohlendioxids in das Kondensat erfolgt.

7. Verfahren zur Reinigung einer Struvit-Ablagerungen aufweisenden Rohrleitung (17, 18), mit den Schritten:
- Erzeugen einer Struvit-Ablagerungen in Rohrleitungen beseitigenden Reinigungsflüssigkeit nach einem der vorhergehenden Ansprüche,
- Einleiten der Reinigungsflüssigkeit in die Rohrleitung (17, 18) und Einwirken lassen der Reinigungsflüssigkeit auf die Struvit-Ablagerungen bis die Struvit-Ablagerungen in der Reinigungsflüssigkeit rückgelöst sind.

8. Verfahren nach Anspruch 7, **gekennzeichnet durch** Ausleiten der die Struvit-Ablagerungen in Lösung aufweisenden Reinigungsflüssigkeit in einen Lagerbehälter (20).

9. Verfahren nach Anspruch 7, **dadurch gekennzeichnet, dass** die die Struvit-Ablagerungen in Lösung aufweisende Reinigungsflüssigkeit in einen Rückgewinnungsbehälter (24) ausgeleitet und das in dieser Flüssigkeit enthaltende Kohlendioxid durch Versetzen dieser Flüssigkeit mit einem Gas unter Erhöhen des pH-Werts zum Ausfällen des Struvits ausgetrieben wird.

10. Verfahren nach Anspruch 9, **dadurch gekennzeichnet, dass** die Struvit depletierte Reinigungsflüssigkeit zur Herstellung einer Struvit-Ablagerungen in Rohrleitungen (17, 18) beseitigenden Reinigungsflüssigkeit gemeinsam mit dem Kondensat oder anstelle des Kondensats behandelt wird.

11. Verfahren nach einem der Ansprüche 9 und 10, **dadurch gekennzeichnet, dass** die die Struvit-Ablagerungen in Lösung aufweisende Reinigungsflüssigkeit in einen Rückgewinnungsbehälter (24) ausgeleitet wird, solange der pH-Wert der Reinigungsflüssigkeit einen vorbestimmten pH-Wert überschreitet.

12. Biogasanlage mit einer ein Kondensat erzeugenden Trocknungseinrichtung (1) zur Trocknung von Rohbiogas (3) und einer für Struvit-Ablagerungen anfälligen Rohrleitung (17, 18),
**gekennzeichnet durch**
einen Behälter (6) zur Aufbereitung des Kondensats zu einer Struvit-Ablagerungen beseitigenden Reinigungsflüssigkeit, mit
- einer Heizeinrichtung (7) zum Sieden des Kondensats,
- einer pH-Sonde zur Messung des pH-Werts des Kondensats, und
- wenigstens einer dem Behälter (6) ein Gas zuführenden Leitung (10, 15), wobei der Behälter (6) zur Aufbereitung des Kondensats (2) mittels Rohrleitungen mit der Trocknungseinrichtung (1) und der für Struvit-Ablagerungen anfälligen Rohrleitung (18) verbunden ist.

13. Biogasanlage nach Anspruch 12, **dadurch gekennzeichnet, dass** die für Struvit-Ablagerungen anfällige Rohrleitung (18) mit einem die in der Reinigungsflüssigkeit rückgelösten Struvit-Ablagerungen aufnehmenden Lagerbehälter (20) verbunden ist.

14. Biogasanlage nach einem der Ansprüche 12 und 13, **gekennzeichnet durch** einen zur Rückgewinnung von Struvit aus der Reinigungsflüssigkeit eingerichteten Rückgewinnungsbehälter (24), der mit der für Struvit-Ablagerungen anfälligen Rohrleitung (17, 18) und dem Behälter (6) verbunden ist, wobei der Rückgewinnungsbehälter (24) eine Belüftungseinrichtung (27, 28) zum Versetzen der gelöste Struvit-Ablagerungen aufweisenden Reinigungsflüssigkeit mit einem Gas zum Erhöhen des pH-Werts zum Ausfällen des Struvits aufweist.

15. Biogasanlage nach einem der Ansprüche 12 bis 14, **dadurch gekennzeichnet, dass** der Rückgewinnungsbehälter (24) einen trichterförmigen Abschnitt (25) zur Aufnahme und Abfuhr aus der Reinigungsflüssigkeit ausgefällten Struvits aufweist.
